# EUROPÄISCHE PATENTSCHRIFT

(11) **EP 0 343 476 B1**
(45) Veröffentlichungstag und Bekanntmachung des Hinweises auf die Patenterteilung: **14.12.1994**
(21) Anmeldenummer: 89108699.3
(22) Anmeldetag: 16.05.1989
(51) Int. Cl.: C07C 275/20, C07D 211/58, C07D 295/20

(54) **Ethylenisch ungesättigte Harnstoffderivate und Verfahren zu ihrer Herstellung**
Ethylenic unsaturated urea derivatives and process for their preparation
Dérivés éthyléniques non saturés d'urée et procédé de leur préparation

(30) Priorität: 21.05.1988 DE 3817468
(43) Veröffentlichungstag der Anmeldung: 29.11.1989
(73) Patentinhaber: HOECHST AKTIENGESELLSCHAFT, 65926 Frankfurt am Main (DE)
(72) Erfinder: Huth, Hans-Ullrich, Dr., D-6073 Egelsbach (DE)

(56) Entgegenhaltungen:
- EP-A- 226 953
- EP-A- 238 166
- EP-A- 243 890
- EP-A- 0 181 281
- EP-A- 0 189 059
- EP-A- 0 201 386
- DE-A- 2 437 639
- GB-A- 761 901
- CHEMICAL ABSTRACTS, BD. 101 (1984), 192568Q.
- Die Makromulekulare Chemie, Suppl. 1, 71-95 (1975).
- Die Makromolekulare Chemie 161, 1-47 (1972).

## Beschreibung

Die Erfindung betrifft ethylenisch ungesättigte Harnstoffderivate, insbesondere Harnstoffderivate mit (Meth)Acrylester- oder (Meth)Acrylamidgruppen, die teilweise interessante Löslichkeiten in Wasser und/oder organischen Lösungsmitteln aufweisen und sich u.a. radikalisch polymerisieren bzw. copolymerisieren lassen, sowie Verfahren zu ihrer Herstellung.

Aus der EP-B 0 003870 sind bereits ethylenisch ungesättigte Harnstoffderivate mit (Meth)Allylgruppen und deren Verwendung als Comonomere bei der Emulsionspolymerisation bekannt. Bei ihrer Verwendung als Monomere zeigen sie jedoch die bei der Allylpolymerisation bekannten Nachteile.

Aus GB-A 761 901 sind (Meth)Acrylestergruppen enthaltende Harnstoffderivate bekannt, die im Harnstoffteil unsubstituiert sind. Diese Verbindungen besitzen eine hohe Wasserlöslichkeit (Vergleichsbeispiel 1).

Weitere Ureido(meth)acrylate sind aus den folgenden Druckschriften bekannt: DE-A 2 437 639, EP-A 0 243 890, EP-A 0 238 166, EP-A 0181 281, EP-A 0 226 953, EP-A 0 189 059, Chemical Abstracts, Bd. 101, 1925 68 q (1984) sowie aus Die Makromolekulare Chemie, Suppl. 1, 71-95 (1975) und Die Makromolekulare Chemie 161, 1-47 (1972). Alle in diesen Druckschriften aufgeführten Harnstoffderivate tragen an dem die (Meth)Acrylatgruppe bindenden Stickstoffatom ein Wasserstoffatom.

Aus der EP-A 0 197 635 sind ethylenisch ungesättigte Harnstoffverbindungen mit (Meth)Acrylesterresten bekannt, deren Harnstoffgruppe an dem der (Meth)Acrylestergruppe benachbarten N-Atom durch H und am anderen N-Atom durch einen größeren organischen Rest mit mindestens 5 C-Atomen substituiert ist. Diese Harnstoffverbindungen zeigen weder in Wasser noch in den meisten organischen Lösungsmitteln nennenswerte Löslichkeiten. Sie können durch Copolymerisation mit α,β-ethylenisch ungesättigten Carbonsäuren und weiteren Comonomeren zur Herstellung von niedrig-viskosen Verdickerdispersionen verwendet werden, die sich durch Einstellung ihres pH-Wertes auf etwa 9 in hochviskose wäßrige Lösungen überführen lassen. Diese Produkte sollen eine bessere Verseifungsbeständigkeit und bessere Elektrolytbeständigkeiten, vergleichsweise zu anderen üblichen Verdickern, besitzen und zur Verbesserung der rheologischen Eigenschaften von wäßrigen Systemen verwendet werden können. Über andere Verwendungen dieser Produkte ist nichts ausgeführt.

Es wurden nun überraschenderweise Harnstoffderivate mit α,β-ethylenisch ungesättigten Carboxy- oder Carbamidresten gefunden, die an dem die Carboxy-/Carbamidgruppe bindunden Stickstoffatom kein Wasserstoffatom tragen, die geringe Löslichkeiten in Wasser und geringe oder mäßige bis gute Löslichkeiten in organischen Lösungsmitteln aufweisen, die polymerisationsfähig und copolymerisationsfähig sind und sich vorteilhaft und vielseitig, beispielsweise zur Herstellung von Polymerisaten oder Copolymerisaten, verwenden lassen.

Gegenstand der Erfindung sind daher ethylenisch ungesättigte Harnstoffderivate der Formel I, 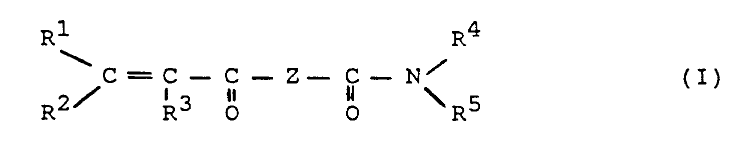
worin die Reste R¹ bis R⁵ und Z folgendes bedeuten:
- R¹, R², R³: , die gleich oder verschieden sein können, = H oder CH₃, 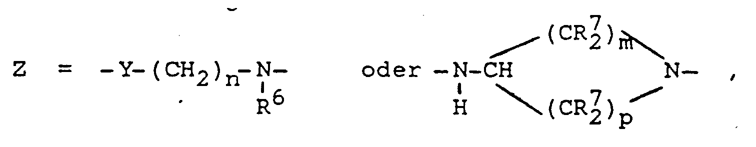
- Y: = Sauerstoff oder NH, n = 2 bis 4, vorzugsweise 2,
- R⁶: = gegebenenfalls substituiertes (C₁-C₄)-Alkyl,
- R⁷: = H oder CH₃ und m, p = jeweils mindestens die Zahl 1, vorzugsweise m + p = 3 bis 5,
- R⁴: = H,
- R⁵: = gegebenenfalls substituiertes (C₁-C₃₀)-Alkyl, vorzugsweise (C₁-C₁₈)-Alkyl, gegebenenfalls substituiertes -(CₖH₂ₖ)-OH mit k = 1 bis 8, vorzugsweise 2 bis 4, gegebenenfalls substituiertes (C₆-C₁₀)-Aryl, gegebenenfalls substituiertes (C₇-C₃₀)-Aralkyl, gegebenenfalls substituiertes (C₅-C₈)-Cycloalkyl, einen gegebenenfalls substituierten 5 bis 7-gliedrigen Heterocyclus.

Bevorzugte Harnstoffderivate sind ferner solche, bei denen in Formel I R¹, R² = H und R³ = CH₃, oder R¹,R² = H, R³ = CH₃ und 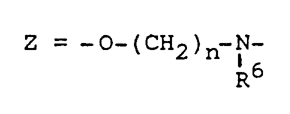
mit R⁶ = -C(CH₃)₃ bedeutet.

Gegenstand der Erfindung ist ferner ein Verfahren zur Herstellung von ethylenisch ungesättigten Harnstoffderivaten der Formel I, 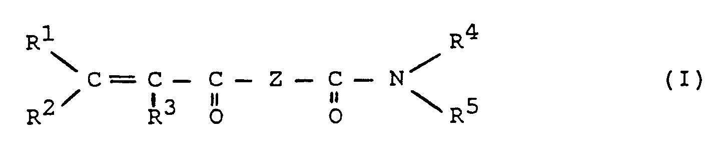
worin R¹ bis R⁵ und Z die vorstehend genannten Bedeutungen haben, durch Umsetzung von Isocyanaten mit
Aminen, indem man
Amine der Formeln II oder III, 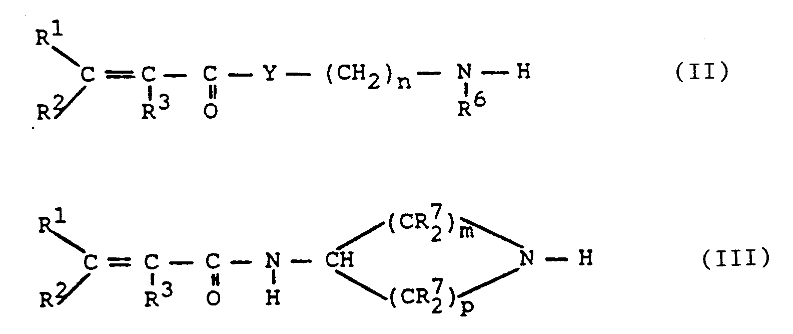
worin R¹, R², R³, R⁶, R⁷, Y, n, m und p die Bedeutung wie in Formel I haben, mit Isocyanaten der Formel IV,

OCN - R⁵ (IV)

worin R⁵ die Bedeutung wie in Formel I hat, umsetzt.

Die Durchführung des Verfahrens erfolgt erfindungsgemäß bevorzugt in der Weise, daß man die Umsetzungen in Substanz oder vorzugsweise in inerten organischen Lösungsmitteln bzw. in unter den Umsetzungsbedingungen sich inert verhaltenden copolymerisationsfähigen ethylenisch ungesättigten Monomeren (Reaktivverdünner) in Abwesenheit von Wasser, vorzugsweise bei Temperaturen zwischen 0°C und Raumtemperatur, oder bei einer höheren Temperatur, vorzugsweise bis zu 60°C, durchführt.

Als inerte organische Lösungsmittel kommen die bei organischen Synthesen mit Isocyanaten in wasserfreiem Medium üblicherweise verwendeten inerten Lösungsmittel zur Anwendung, sofern sie u.a. die Erfordernisse hinsichtlich Lösewirkung und Siedebereich erfüllen können.

Bevorzugte inerte Lösungsmittel sind z.B. Toluol, Tetrahydrofuran (THF), Ethylacetat und Hexan.

Bevorzugt sind ferner auch sogenannte Reaktivverdünner, das sind copolymerisationsfähige Monomere, die sich unter den Synthesebedingungen der erfindungsgemäßen Harnstoffgruppenbildung inert verhalten, sich aber später unter geeigneten Polymerisationsbedingungen mit den erfindungsgemäßen ungesättigten Harnstoffderivaten der Formel I copolymerisieren lassen. Bevorzugte inerte Reaktivverdünner sind demzufolge z.B. (Meth)Acrylsäureester, Styrol und Vinylester, wobei man bei deren Verwendung vorzugsweise unterhalb der Sättigungskonzentration der Reaktanten arbeitet.

Gegebenenfalls wird bei der erfindungsgemäßen Isocyanataddition ein Katalysator mitverwendet, u.a. um die Reaktionstemperatur möglichst niedrig halten zu können, was insbesondere bei der Verwendung von Reaktivverdünnern vorteilhaft sein kann. Bevorzugte Katalysatoren sind organische Zinnverbindungen, die vorzugsweise in einem inerten organischen Lösungsmittel gelöst eingesetzt werden. Besonders bevorzugt ist die Verwendung von Dibutylzinndilaurat.

Als Amine der Formel II werden bevorzugt solche verwendet, bei denen in Formel II R¹, R², R³ = H oder CH₃, vorzugsweise R¹, R² = H und R³ = CH₃, Y = 0, n = 2 bis 4, vorzugsweise 2, R⁶ = (C₁-C₄)-Alkyl, insbesondere Butyl, besonders bevorzugt tert.-Butyl, bedeutet.

Als Amine der Formel III werden bevorzugt solche verwendet, bei denen in Formel III R¹, R², R³ die Bedeutung wie in Formel II haben, R⁷ = H oder CH₃ und m + p = 3 bis 5, vorzugsweise m = 2 und p = 2 entsprechend einem Piperidinrest, bedeuten. Besonders bevorzugte Verbindungen der Formel III sind solche, bei denen mindestens ein Rest R⁷, insbesondere vier Reste R⁷, vorzugsweise vier Reste R⁷ in 2- und 6-Position des Heterocyclus, CH₃ bedeuten.

Als Isocyanate der Formel IV können insbesondere die üblichen bekannten Alkyl-, Aryl-, Aralkyl- und Cycloalkylmonoisocyanate verwendet werden. Besonders bevorzugt sind z. B. Methylisocyanat, Ethylisocyanat, Butylisocyanat, Nonylisocyanat, Oktadecylisocyanat, Phenylisocyanat, 3-Chlorphenylisocyanat, 3-Toluylisocyanat und Cyclohexylisocyanat.

Die erfindungsgemäß hergestellten Verbindungen der Formel I werden in den meisten Fällen unmittelbar in kristalliner Form erhalten und können, gegebenfalls nach Absaugen von mitverwendeten Lösungsmitteln, im allgemeinen ohne weitere Reinigung und Trocknung als Zwischenprodukte weiter verwendet werden. In manchen Fällen fallen Verbindungen der Formel I auch in flüssig-viskoser Form an und können als Zwischenprodukte ebenfalls direkt weiter verwendet werden. Selbstverständlich können die bei dem erfindungsgemäßen Syntheseverfahren anfallenden rohen Reaktionsprodukte auch nach üblichen Methoden, wie z. B. Eluieren, Umkristallisieren, Umfällen und/oder Abdestillieren flüchtiger Bestandteile gegebenenfalls unter vermindertem Druck, gereinigt und die Verbindungen der Formel I in chemisch reiner Form gewonnen werden.

Ihr Löslichkeitsverhalten eröffnet den Harnstoffderivaten der Formel I interessante Verwendungsmöglichkeiten, insbesondere als Comonomere bei Polymerisationen. Es wurde diesbezüglich überraschenderweise gefunden, daß vorzugsweise durch radikalisch initiierte Emulsions- oder Suspensionspolymerisation hergestellte Dispersionspolymerisate auf der Basis von ethylenisch ungesättigten Monomeren, die mindestens 1 Gew.-% Monomereinheiten aus Harnstoffderivaten der Formel I der vorliegenden Erfindung enthalten, unerwartet vorteilhafte Eigenschaften besitzen. Diese Polymerisate bzw. Copolymerisate, ihre Verwendbarkeit, insbesondere in Form ihrer wäßrigen Dispersionen, sowie ihre Herstellung unter Verwendung monomerer Harnstoffderivate der Formel I der vorliegenden Erfindung als Ausgangsprodukte sind Gegenstand der am gleichen Tag eingereichten Patentanmeldung EP-A-0343477, auf die hiermit Bezug genommen wird.

Die Erfindung wird durch die nachstehenden Beispiele näher erläutert.

### Vergleichs beispiel 1

### Herstellung der Formel I-Verbindung

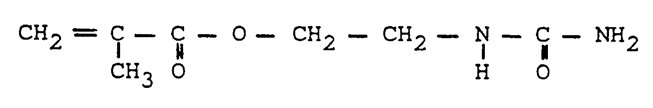

In einem Vierhalskolben mit Rührer, Thermometer, Gaseinleitungsrohr und Rückflußkühler mit CaCl₂-Endrohr werden 1,2 l trockenes n-Hexan, 155,1 g (1 Mol) Isocyanatoethylmethacrylat und 0,1 ml Dibutylzinndilaurat vorgelegt. Anschließend wird bei einer Temperatur zwischen 0 und 5°C mindestens 1 Mol NH₃ als trockenes Ammoniakgas unter Rühren unmittelbar unter die Flüssigkeitsoberfläche in die Reaktionsmischung eingeleitet. Nach einiger Reaktionszeit trübt sich das Reaktionsgemisch und die gewünschte ethylenisch ungesättigte Harnstoffverbindung der obengenannten Formel I beginnt auszukristallisieren. Nach einer Reaktionszeit von ca. 2,5 Stunden wird das Reaktionsprodukt vom flüssigen Anteil abgesaugt, dreimal mit 100 ml n-Hexan gewaschen und unter Vakuum getrocknet. Die Ausbeute an kristallinem Reaktionsprodukt der obengenannten Formel I beträgt 83,1 g (48,3 % der Theorie), sein Schmelzpunkt liegt bei 74-77°C. Das Produkt ist leicht löslich in Wasser und gut löslich in einigen organischen Lösungsmitteln, wie z. B. Toluol, Acrylester, Styrol. Das IR- und das ¹H-NMR-Spektrum stehen mit der Struktur der Harnstoffverbindung der obengenannten Formel I im Einklang.

### Beispiel 2

### Herstellung der Formel I-Verbindung

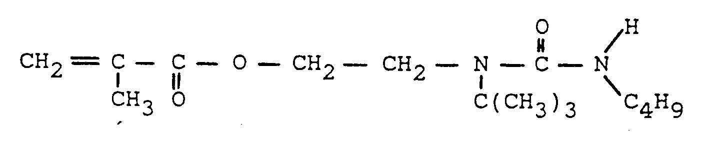

Es wird die in Beispiel 1 beschriebene Apparatur mit der Abänderung verwendet, daß das Gaseinleitungsrohr durch einen Tropftrichter ersetzt wurde. Es werden in dem Vierhalskolben 92.5 g (0,5 Mol) tert.- Butylaminoethylmethacrylat und 1 Tropfen Dibutylzinndilaurat (Sn-Katalysator), gelöst in 100 ml Toluol, vorgelegt und bei Raumtemperatur unter Rühren 49.6 g (0,5 Mol) Butylisocyanat bei einer Temperatur zwischen Raumtemperatur und 30°C in die Vorlage getropft. Nach beendeter Reaktion läßt man ca. 1 Stunde nachreagieren. Die Ausbeute an flüssigem, hochviskosem, gelblich gefärbtem Reaktionsprodukt der obengenannten Formel I beträgt 136,2 g (95,9 % der Theorie). Das Produkt ist mit verschiedenen organischen Lösungsmitteln gut mischbar und mit Wasser nur geringfügig mischbar. Das IR- und das ¹H-NMR-Spektrum stehen mit der Struktur der Harnstoffverbindung gemäß der obengenannten Formel I im Einklang.

### Beispiel 3

### Herstellung der Formel I-Verbindung

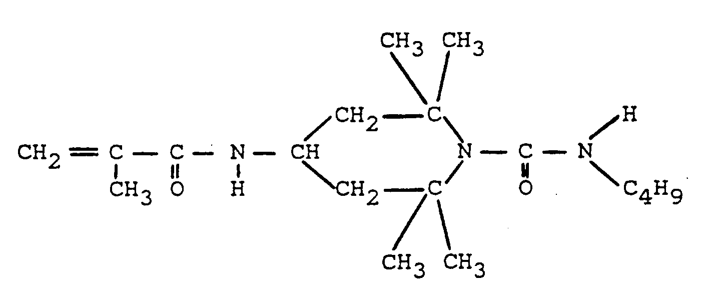

Es wird die in Beispiel 2 beschriebene Apparatur verwendet und es wird wie in Beispiel 2 beschrieben, jedoch mit folgender Abänderung verfahren. Es werden 48,9 g (0,2 Mol) 4-Methacrylamido-2,2,6,6-tetramethylpiperidin (MATMP) in Toluol mit dem Sn-Katalysator vorgelegt und 19,8 g (0,2 Mol) n-Butylisocyanat bei 40°C zudosiert. Die Ausbeute an festem Reaktionsprodukt der obengenannten Formel I beträgt 36 g (52,4 % der Theorie); Schmelzpunkt: 128°C. Das Produkt zeigt nur geringe Löslichkeit sowohl in Wasser als auch in organischen Lösungsmitteln. Das IR- und das ¹H-NMR-Spektrum stehen mit der Struktur der Harnstoffverbindung gemäß der obengenannten Formel I im Einklang.

### Beispiel 4

### Herstellung der Formel I-Verbindung

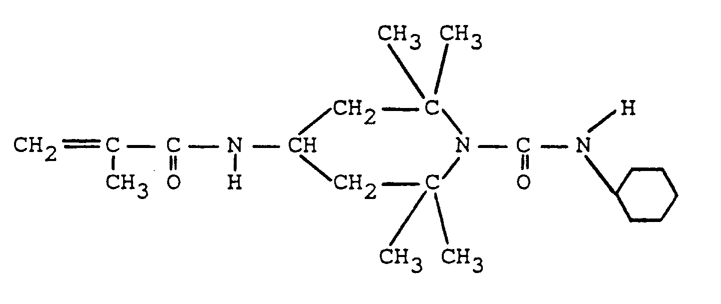

Es wird die in Beispiel 2 beschriebene Apparatur verwendet und es wird wie in Beispiel 2 beschrieben, jedoch mit folgender Abänderung verfahren. Es werden 122,2 g (0,5 Mol) MATMP (vgl. Beispiel 3) in Toluol mit dem Sn-Katalysator vorgelegt und 62,5 g (0,5 Mol) Cyclohexylisocyanat bei 50°C zudosiert. Die Ausbeute an festem Reaktionsprodukt der obengenannten Formel I beträgt 103,5 g (56 % der Theorie); Schmelzpunkt: 129°C. Das Produkt zeigt nur geringe Löslichkeit sowohl in Wasser als auch in organischen Lösungsmitteln. Das IR- und das ¹H-NMR-Spektrum stehen mit der Struktur der Harnstoffverbindung gemäß der obengenannten Formel I im Einklang.

### Beispiel 5

### Herstellung der Formel I-Verbindung

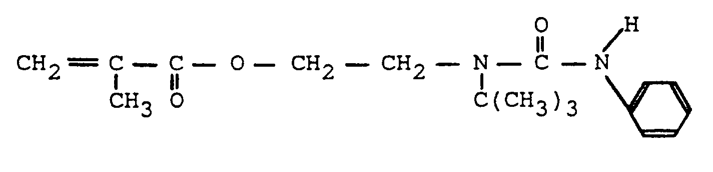

Es wird die in Beispiel 2 beschriebene Apparatur verwendet und es wird wie in Beispiel 2 beschrieben, jedoch mit folgender Abänderung verfahren. Es werden 92,5 g (0,5 Mol) tert.-Butylaminoethylmethacrylat in Toluol mit dem Sn-Katalysator vorgelegt und 59,6 g (0,5 Mol) Phenylisocyanat bei 50°C zudosiert. Die Ausbeute an festem Reaktionsprodukt der obengenannten Formel I beträgt 150,6 g (99 % der Theorie); Schmelzpunkt: 63°C. Das Produkt ist leicht löslich in verschiedenen organischen Lösungsmitteln und wenig löslich in Wasser. Das IR- und das ¹H-NMR-Spektrum stehen mit der Struktur der Harnstoffverbindung gemaß der obengenannten Formel I im Einklang.

## Patentansprüche (Patentansprüche für folgende(n) Vertragsstaat(en): AT, BE, CH, DE, FR, GB, GR, IT, LI, NL, SE)

1. Ethylenisch ungesättigte Harnstoffderivate der Formel I, 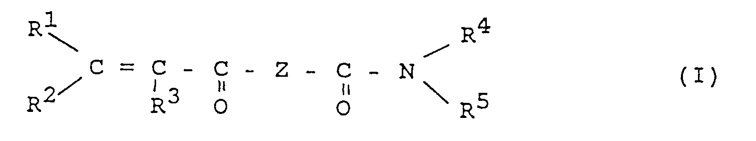 worin die Reste R¹ bis R⁵ und Z folgendes bedeuten:
R¹, R², R³, die gleich oder verschieden sein können, = H oder CH₃, 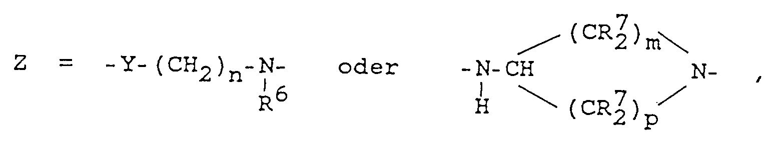
Y = Sauerstoff oder NH, n = 2 bis 4, vorzugsweise 2,
R⁶ = gegebenenfalls substituiertes (C₁-C₄)-Alkyl,
R⁷ = H oder CH₃ und m, p = jeweils mindestens die Zahl 1, vorzugsweise m + p = 3 bis 5,
R⁴ = H,
R⁵ = gegebenenfalls substituiertes (C₁-C₃₀)-Alkyl, vorzugsweise (C₁-C₁₈)-Alkyl, gegebenenfalls substituiertes -(CₖH₂ₖ)-OH mit k = 1 bis 8, vorzugsweise 2 bis 4, gegebenenfalls substituiertes (C₆-C₁₀)-Aryl, gegebenenfalls substituiertes (C₇-C₃₀) Aralkyl, gegebenenfalls substituiertes (C₅-C₈)-Cycloalkyl, einen gegebenenfalls substituierten 5- bis 7-gliedrigen Heterocyclus.

2. Harnstoffderivate nach Anspruch 1, dadurch gekennzeichnet, daß in Formel I R¹, R² = H und R³ = CH₃, oder R¹, R² = H, R³ = CH₃ und 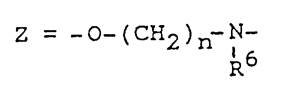 mit R⁶ = -C(CH₃)₃ bedeutet.

3. Verfahren zur Herstellung von ethylenisch ungesättigten Harnstoffderivaten der Formel I nach einem oder mehreren der Ansprüche 1 und 2, 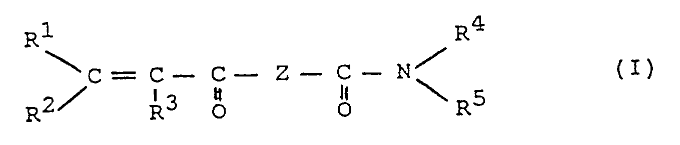 worin R¹ bis R⁵ und Z die Bedeutung wie in Formel I in Ansprüchen 1 bis 3 haben, durch Umsetzung von Isocyanaten mit Aminen, dadurch gekennzeichnet, daß man Amine der Formeln II oder III, 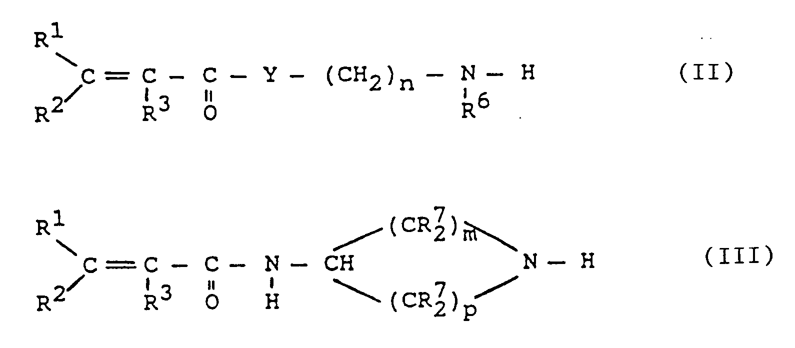 worin R¹, R², R³, R⁶, R⁷, Y, n, m und p die Bedeutung wie in Formel I haben, mit Isocyanaten der Formel IV,
OCN - R⁵ (IV)
worin R⁵ die Bedeutung wie in Formel I hat, umsetzt.

4. Verfahren nach Anspruch 3, dadurch gekennzeichnet, daß man die Umsetzungen in Substanz oder vorzugsweise in inerten organischen Lösungsmitteln bzw. in unter den Umsetzungsbedingungen sich inert verhaltenden copolymerisationsfähigen ethylenisch ungesättigten Monomeren in Abwesenheit von Wasser, vorzugsweise bei Temperaturen zwischen 0°C und Raumtemperatur, oder bei einer höheren Temperatur, durchführt.

## Patentansprüche (Patentansprüche für folgende(n) Vertragsstaat(en): ES)

1. Verfahren zur Herstellung von ethylenisch ungesättigten Harnstoffderivaten der Formel I, 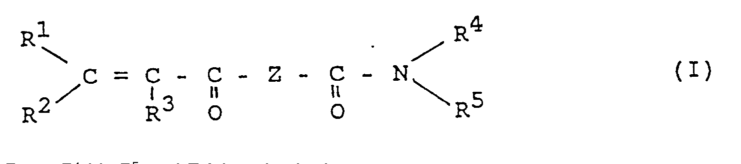 worin die Reste R¹ bis R⁵ und Z folgendes bedeuten:
R¹, R², R³, die gleich oder verschieden sein können, = H oder CH₃, 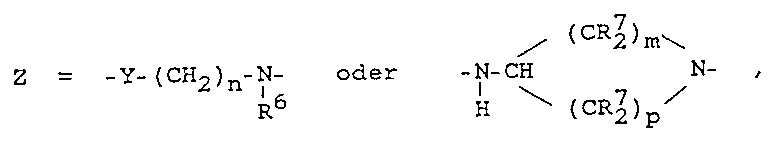
Y = Sauerstoff oder NH, n = 2 bis 4, vorzugsweise 2,
R⁶ = gegebenenfalls substituiertes (C₁-C₄)-Alkyl,
R⁷ = H oder CH₃ und m, p = jeweils mindestens die Zahl 1, vorzugsweise m + p = 3 bis 5,
R⁴ = H,
R⁵ = gegebenenfalls substituiertes (C₁-C₃₀)-Alkyl, vorzugsweise (C₁-C₁₈)-Alkyl, gegebenenfalls substituiertes -(CₖH₂ₖ)-OH mit k = 1 bis 8, vorzugsweise 2 bis 4, gegebenenfalls substituiertes (C₆-C₁₀)-Aryl, gegebenenfalls substituiertes (C₇-C₃₀) Aralkyl, gegebenenfalls substituiertes (C₅-C₈)-Cycloalkyl, einen gegebenenfalls substituierten 5- bis 7-gliedrigen Heterocyclus durch Umsetzung von Isocyanaten mit Aminen, dadurch gekennzeichnet, daß man
Amine der Formeln II oder III, 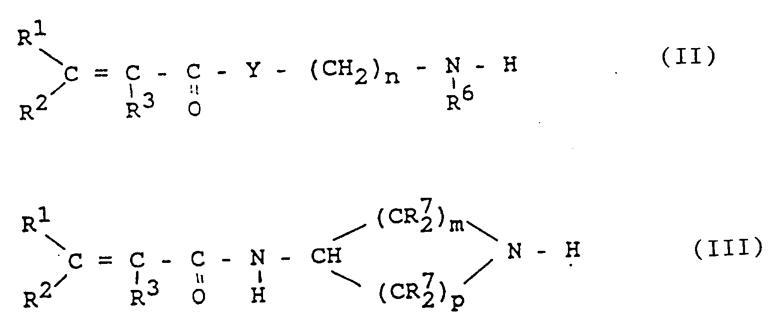 worin R¹, R², R³, R⁶, R⁷, Y, n, m und p die Bedeutung wie in Forrel I haben, mit Isocyanaten der Formel IV,
OCN - R⁵ (IV)
worin R⁵ die Bedeutung wie in Formel I hat, umsetzt.

2. Verfahren nach Anspruch 1, dadurch gekennzeichnet, daß in Formel I R¹, R² = H und R³ = CH₃, oder R¹, R² = H, R³ = CH₃ und 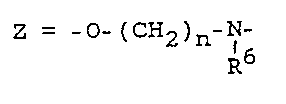 mit R⁶ = -C(CH₃)₃ bedeutet.

3. Verfahren nach einem oder mehreren der Ansprüche 1 und 2, dadurch gekennzeichnet, daß man die Umsetzungen in Substanz oder vorzugsweise in inerten organischen Lösungsmitteln bzw. in unter den Umsetzungsbedingungen sich inert verhaltenden copolymerisationsfähigen ethylenisch ungesättigten Monomeren in Abwesenheit von Wasser, vorzugsweise bei Temperaturen zwischen 0°C und Raumtemperatur, oder bei einer höheren Temperatur, durchführt.

## Claims (Claims for the following Contracting State(s): AT, BE, CH, DE, FR, GB, GR, IT, LI, NL, SE)

1. Ethylenically unsaturated urea derivatives of the formula I; 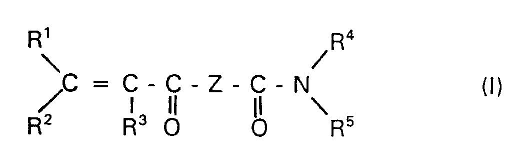 in which the radicals R¹ to R⁵ and Z signify the following:
R¹, R², R³, which may be identical or different, = H or CH₃ 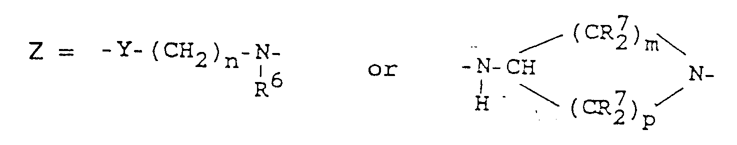
Y = oxygen or NH, n = 2 to 4, preferably 2,
R⁶ = an optionally substituted (C₁-C₄)-alkyl,
R⁷ = H or CH₃ and m, p = in each case at least the number 1, preferably m + p = 3 to 5,
R⁴ = H,
R⁵ = optionally substituted (C₁-C₃₀)-alkyl, preferably (C₁-C₁₈)-alkyl, an optionally substituted -(CₖH₂ₖ)- OH with k = 1 to 8, preferably 2 to 4, an optionally substituted (C₆-C₁₀)-aryl, an optionally substituted (C₇-C₃₀)-aralkyl, an optionally substituted (C₅-C₈)-cycloalkyl or an optionally substituted 5- to 7-membered heterocycle.

2. Urea derivates as claimed in claim 1, wherein in formula I R¹, R² = H and R³ = CH₃, or R¹, R² = H, R³ = CH₃ and 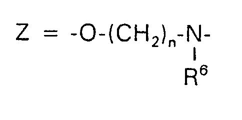 with R⁶ = -C(CH₃)₃.

3. A process for the preparation of ethylenically unsaturated urea derivatives of the formula I as claimed in claims 1 and 2 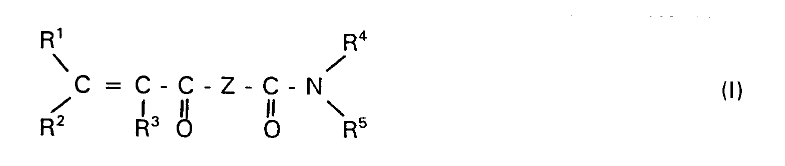 in which R¹ to R⁵ and Z have the meaning as in formula I in claims 1 and 2, by reaction of isocyanates with amines, which comprises reacting amines of the formulae II or III 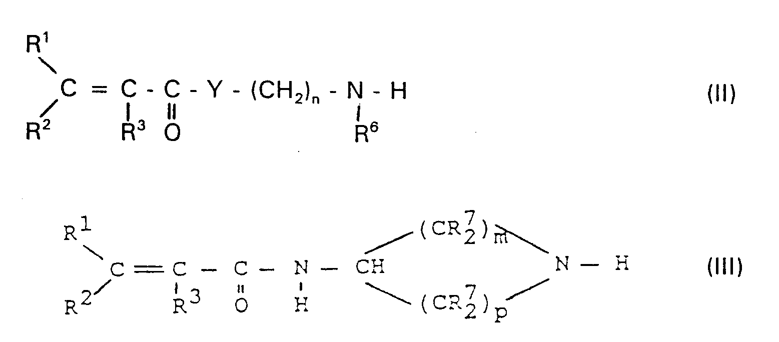 in which R¹, R², R³, R⁶, R⁷, Y, n, m and p have the meaning as in formula I with isocyanates of the formula IV
OCN-R⁵ (IV)
in which R⁵ has the meaning as in formula I.

4. The process as claimed in claim 3, wherein the reactions are carried out in bulk or preferably in inert organic solvents or in copolymerizable ethylenically unsaturated monomers which remain inert under the reaction conditions, in the absence of water, preferably at temperatures between 0°C and room temperature, or at a higher temperature.

## Claims (Claims for the following Contracting State(s): ES)

1. Process for the preparation of ethylenically unsaturated urea derivatives of the formula I, 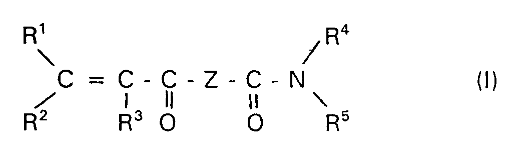 in which the radicals R¹ to R⁵ and Z signify the following:
R¹, R², R³, which may be identical or different, = H or CH₃ 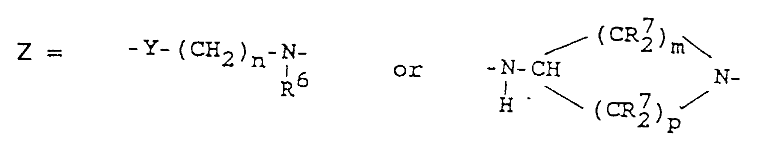
Y = oxygen or NH, n = 2 to 4, preferably 2,
R⁶ = an optionally substituted (C₁-C₄)-alkyl,
R⁷ = H or CH₃ and m, p = in each case at least the number 1, preferably m + p = 3 to 5,
R⁴ = H,
R⁵ = optionally substituted (C₁-C₃₀)-alkyl, preferably (C₁-C₁₈)-alkyl, an optionally substituted -(CₖH₂ₖ)-OH with k = 1 to 8, preferably 2 to 4, an optionally substituted (C₆-C₁₀)-aryl, an optionally substituted (C₇-C₃₀)-aralkyl, an optionally substituted (C₅-C₈)-cycloalkyl or an optionally substituted 5- to 7-membered heterocycle, by reaction of isocyanates with characterized in that amines of the formulae II or III. 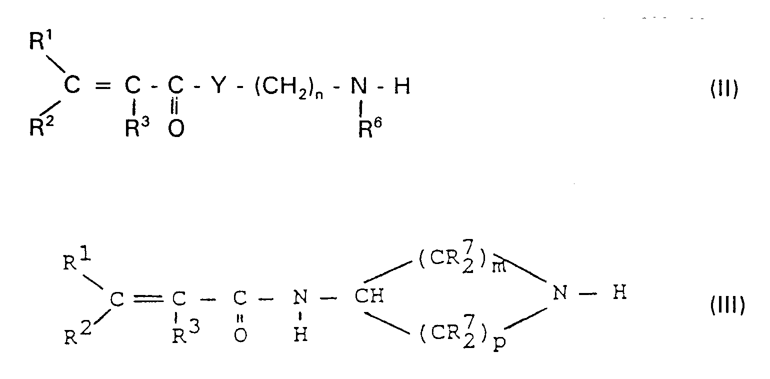
in which R¹, R², R³, R⁶, R⁷, Y, n, m and p have the meaning as in formula I are reacted with isocyanates of the formula IV
OCN-R⁵ (IV)
in which R⁵ has the meaning as in formula I.

2. Urea derivates as claimed in claim 1, wherein in formula I R¹, R² = H and R³ = CH₃, or R¹, R² = H, R³ = CH₃ and 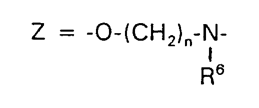 with R⁶ = -C(CH₃)₃.

3. The process as claimed in one or both of claims 1 and 2, wherein the reactions are carried out in bulk or preferably in inert organic solvents or in copolymerizable ethylenically unsaturated monomers which remain inert under the reaction conditions, in the absence of water, preferably at temperatures between 0°C and room temperature, or at a higher temperature.

## Revendications (Revendications pour l'(les) Etat(s) contractant(s) suivant(s): AT, BE, CH, DE, FR, GB, GR, IT, LI, NL, SE)

1. Dérivés de l'urée à insaturation éthylénique de formule I, 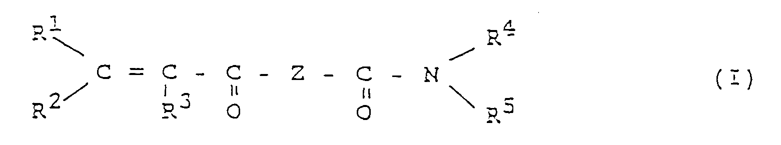 dans laquelle les radicaux R¹ à R⁵ et Z ont les significations suivantes :
R¹, R², R³, qui peuvent être identiques ou différents, représentent H ou CH₃, 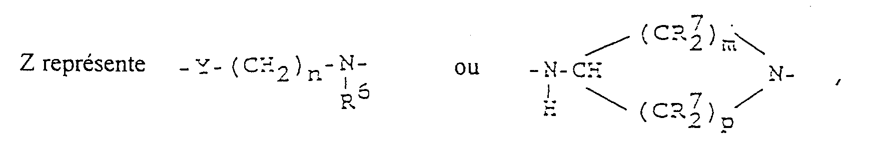 Y représente l'oxygène ou NH, n est de 2 à 4, de préférence 2,
R⁶ représente un alkyle en C₁-C₄ éventuellement substitué,
R⁷ représente H ou CH₃ et m, p représentent chaque fois au moins le nombre 1, de préférence m+p = 3 à 5,
R⁴ représente H,
R⁵ représente un alkyle en C₁-C₃₀ éventuellement substitué, de préférence un alkyle en C₁-C₁₈, -(CₖH₂ₖ)-OH éventuellement substitué avec k ayant la valeur de 1 à 8, de préférence de 2 à 4, un aryle en C₆-C₁₀ éventuellement substitué, un aralkyle en C₇-C₃₀ éventuellement substitué, un cycloalkyle en C₅-C₈ éventuellement substitué, un hétérocycle ayant de 5 à 7 chaînons, éventuellement substitué.

2. Dérivés de l'urée selon la revendication 1, caractérisés en ce que, dans la formule I, R¹, R² représentent H et R³ représente CH₃, ou R¹, R² représentent H, R³ représente CH₃ et 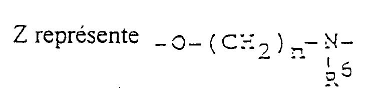 avec R⁶ = -C(CH₃)₃.

3. Procédé pour la préparation des dérivés de l'urée à insaturation éthylénique de formule I selon une ou plusieurs des revendications 1 et 2, 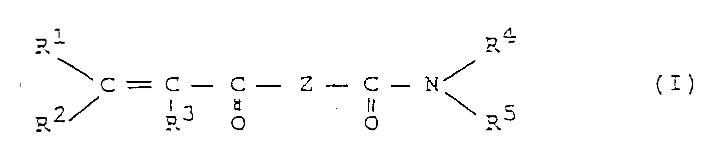 dans laquelle R¹ à R⁵ et Z ont la même signification que celle de la formule I dans les revendications 1 et 2 par réaction d'isocyanates avec des amines, caractérisé en ce que l'on fait réagir des amines de formules II ou III, 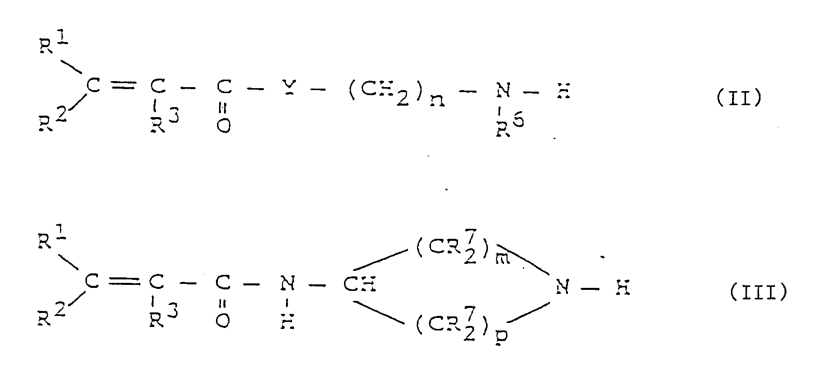 dans lesquelles R¹, R², R³, R⁶, R⁷, Y, n, m et p ont la signification donnée dans la formule I, avec des isocyanates de formule IV,
OCN - R⁵ (IV)
dans laquelle R⁵ a la même signification que dans la formule I.

4. Procédé selon la revendication 3, caractérisé en ce que l'on effectue les réactions en masse ou de préférence dans des solvants organiques inertes ou dans des monomères à insaturation éthylénique susceptibles de copolymérisation, qui se comportent de façon inerte dans les conditions de la réaction en l'absence d'eau, de préférence à des températures entre 0°C et la température ambiante, ou à une température plus élevée.

## Revendications (Revendications pour l'(les) Etat(s) contractant(s) suivant(s): ES)

1. Procédé pour préparer des dérivés de l'urée à insaturation éthylénique de formule I, 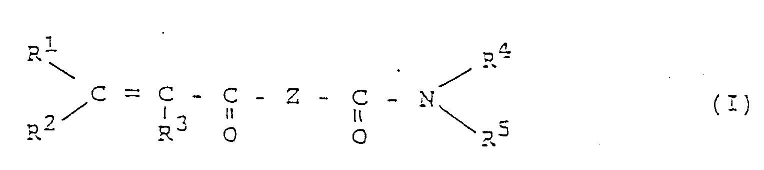 dans laquelle les radicaux R¹ à R⁵ et Z ont les significations suivantes :
R¹, R², R³, qui peuvent être identiques ou différents, représentent H ou CH₃, 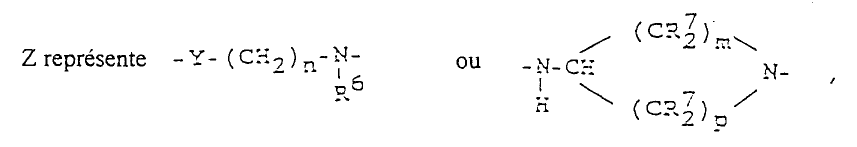 Y représente l'oxygène ou NH, n est de 2 à 4, de préférence 2,
R⁶ représente un alkyle en C₁-C₄ éventuellement substitué,
R⁷ représente H ou CH₃ et m, p représentent chaque fois au moins le nombre 1, de préférence m+p = 3 à 5,
R⁴ représente H,
R⁵ représente un alkyle en C₁-C₃₀ éventuellement substitué, de préférence un alkyle en C₁-C₁₈, -(CₖH_{2K})-OH éventuellement substitué avec k ayant la valeur de 1 à 8, de préférence de 2 à 4, un aryle en C₆-C₁₀ éventuellement substitué, un aralkyle en C₇-C₃₀ éventuellement substitué, un cycloalkyle en C₅-C₈ éventuellement substitué, un hétérocycle ayant de 5 à 7 chaînons, éventuellement substitué,
par réaction d'isocyanates avec des amines, caractérisé en ce que l'on fait réagir des amines des formules II ou III, 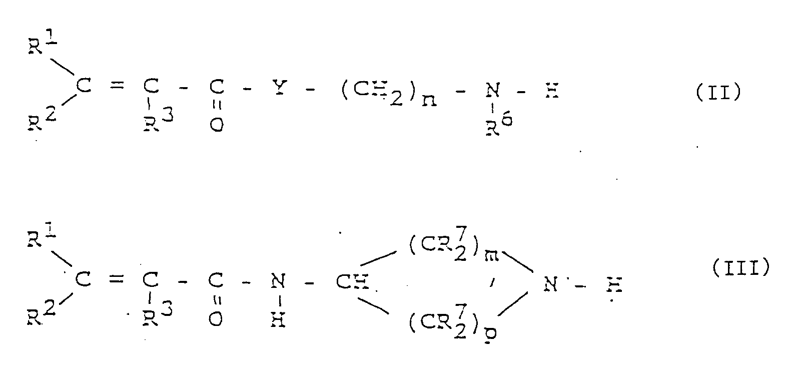 dans lesquelles R¹, R², R³, R⁶, R⁷, Y, n, m et p ont la signification donnée dans la formule I, avec des isocyanates de formule IV,
OCN - R⁵ (IV)
dans laquelle R⁵ a la même signification que dans la formule I.

2. Procédé selon la revendication 1, caractérisé en ce que, dans la formule I, R¹, R² représentent H et R³ représente CH₃, ou R¹, R² représentent H, R³ représente CH₃ et 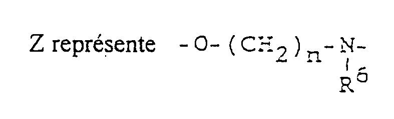 avec R⁶ = -C(CH₃)₃.

3. Procédé selon une ou plusieurs des revendications 1 et 2, caractérisé en ce que l'on effectue les réactions en masse ou de préférence dans des solvants organiques inertes ou dans des monomères à insaturation éthylénique susceptibles de copolymérisation, qui se comportent de façon inerte dans les conditions de la réaction en l'absence d'eau, de préférence à des températures entre 0°C et la température ambiante, ou à une température plus élevée.
